(19) Europäisches Patentamt
European Patent Office
Office européen des brevets

(11) **EP 4 119 039 A1**

(12) **EUROPEAN PATENT APPLICATION**

(43) Date of publication:
**18.01.2023   Bulletin 2023/03**

(21) Application number: **21185843.6**

(22) Date of filing: **15.07.2021**

(51) International Patent Classification (IPC):
**A61B 5/00** (2006.01)        **A61B 5/389** (2021.01)

(52) Cooperative Patent Classification (CPC):
**A61B 5/4519; A61B 5/389**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **CHAKRABARTI, Biswaroop
5656 AE Eindhoven (NL)**

• **BERA, Deep
5656 AE Eindhoven (NL)**
• **HIWALE, Sujitkumar
5656 AE Eindhoven (NL)**
• **KATIPALLY, Karthik Raj
5656 AE Eindhoven (NL)**
• **JOHNSON, Mark Thomas
5656 AE Eindhoven (NL)**

(74) Representative: **Philips Intellectual Property &
Standards
High Tech Campus 52
5656 AG Eindhoven (NL)**

(54) **MUSCLE FIBER SENSING USING ELECTROMYOGRAPHY**

(57)     A means is provided for mapping locations of individual muscle fibers of each of one or more single motor units based on use of TENS for controlled recruitment of motor units, and based on an array of sEMG electrodes placed around the relevant anatomical location. Activation of individual muscle fibers can be detected as individual spikes in the signal at each respective sEMG electrode. The time of arrival of each spike in each sEMG electrode signal can be used to estimate a radial distance of the corresponding muscle fiber from the electrode. From this, triangulation of the locations of each muscle fiber can be performed.

Location of muscle fibre (i)

$c\ (x_c,\ y_c)$

$a\ (x_a,\ y_a)$

$b\ (x_b,\ y_b)$

$d_{ai}$

$d_{bi}$

$$(x-x_a)^2 + (y-y_a)^2 = d_{ai}^2$$
$$(x-x_b)^2 + (y-y_b)^2 = d_{bi}^2$$
$$(x-x_c)^2 + (y-y_c)^2 = d_{ci}^2$$

FIG. 2

EP 4 119 039 A1

**Description**

FIELD OF THE INVENTION

The present invention relates to sensing of muscle fiber activity using Electromyography, and in particular using non-invasive surface Electromyography.

BACKGROUND OF THE INVENTION

Movements in limbs are effected by muscles commanded by the central nervous system through nerves. Movements usually occur around joints between bones. Synovial joints (e.g. shoulder, elbow, wrist, hip, knee and elbow) typically allow movement in multiple planes e.g. flexion/extension, or abduction/adduction. Ball and socket joints, e.g. shoulder and hip, allow movement in three orthogonal planes, while saddle or condyloid joints have a more limited range of movements. There exists a specific muscle or a group of muscles responsible for each movement around a specific joint. For example, at the elbow joint, flexion is effected by biceps brachii while extension is effected by triceps brachii.

The tone of a muscle refers to a state of contraction of a muscle at resting state. In healthy individuals (without any neuromuscular disease or injury), muscles have a normal tone. However, in the presence of pathology, muscle tone increases. The increase in tone is a defensive strategy of the body to limit movement, which has the benefit of reducing pain and further injury. This phenomenon is known as muscle guarding and is one of the causes of reversible stiffness and soreness at or near a joint

Stiffness around a joint may result from joint pathology (e.g. arthritis) or from muscular pathology. Various conditions can present as joint pain/stiffness, such as joint pathologies, inflammatory and degenerative arthritis, traumatic injury to the joints, bursitis, or injury (e.g. meniscus tear, ligament injury, tendinitis, muscular pain/spasm, or fracture near a joint). It may be difficult for a patient or a less qualified caregiver to diagnose the exact cause of pain or stiffness. If the origin of the problem is at the joint (e.g. arthritis, or haemarthrosis), movement is typically painful in all directions and the guarding symmetric. If the origin of the problem is a muscular pathology, this presents as asymmetric guarding.

Electromyography (EMG) is an established technique for diagnosing various neuromuscular pathologies. Reference is made to the following paper which outlines methods for using EMG to examine neuromuscular pathologies : J. Weiss, L. D. Weiss, J. K. Silver, and D. J. Dowling," Easy EMG: a guide to performing nerve conduction studies and electromyography". 2016.

EMG is sometimes combined with nerve conduction studies. In typical usage, EMG employs needle electrodes. The number of electrodes used is typically minimized to avoid patient discomfort. Needleless non-invasive EMG techniques also exist based on use of surface electrodes (sEMG). Surface EMG assesses muscle function by recording muscle activity from the surface above the muscle on the skin. Surface EMG can be recorded by a pair of electrodes or by a more complex array of multiple electrodes. More than one electrode is needed because EMG recordings display the potential difference (voltage difference) between two separate electrodes. However, clinical utility of sEMG is constrained due to difficulty of interpreting results due to variability of skin impedance. Signal-to-noise ratio can also be lower than needle EMG. Techniques have been developed to better interpret surface EMG data.

In general, when a muscle contracts, a plurality of motor units (MUs) are recruited. Analysis of surface EMG readings requires decomposition of acquired signals into different motor unit signal components. Different methods exist for achieving this. Existing methods may be classified into overdetermined and underdetermined approaches. In overdetermined approaches, more sEMG electrodes are employed than motor units expected to be recruited and single motor unit spikes may then be triangulated. This approach assumes that only a small number of motor unit spikes occur simultaneously. In the underdetermined approach, a potential field distribution generated by the plurality of motor units is computed in a modelled volume. The number of recruited MUs may greatly exceed the number of electrodes used. The solutions are less exact, but the method can be used to reconstruct muscular activity in any setting; even when multiple muscles at different depths are active.

One example technique for decomposing sEMG signals into the constituent MUs action potential trains has been described in the following paper: C. J. De Luca, A. Adam, R. Wotiz, L. D. Gilmore, and S. H. Nawab, "Decomposition of Surface EMG Signals," Journal of Neurophysiology, vol. 96, no. 3, pp. 1646-1657, Sep. 2006.

In this example technique, a surface sensor array was used to collect four channels of differentially amplified EMG signals. Decomposition was achieved using a set of algorithms replying on a specially developed knowledge-based Artificial Intelligence framework. In automatic mode, accuracy ranged from 75% to 91%.

Decomposition is typically based on identifying motor unit contributions. However, determining contributions of individual muscle fibers has not yet been achieved in a practical and reliable way. Decomposition of individual muscle fiber activity would be of great clinical benefit in diagnosing neuromuscular pathologies.

There are two main types of muscle fibers: type 1 and type 2. Different muscles of the body have different ratios of these fiber types. Type 1 are known as slow-twitch fibers. Type 2 are known as fast-twitch fibers. This refers to the speed at which the different fiber types contract relative to others. These main types can be further broken down based on how the fibers produce adenosine triphosphate (ATP). On this basis, there can be identified three main types of skeletal muscle fibers:

- Slow oxidative (SO) or Slow twitch fibers contract relatively slowly and use aerobic respiration (oxygen and glucose) to produce ATP.
- Fast oxidative (FO) or fast twitch fibers have fast contractions and primarily use aerobic respiration. Since these are able to switch to anaerobic respiration (glycolysis), these can fatigue more quickly than SO fibers.
- Fast glycolytic (FG) fibers have fast contractions and primarily use anaerobic glycolysis. The FG fibers fatigue more quickly than the others.

Most skeletal muscles in a human contain(s) all three types, although in varying proportions.

The speed of contraction is dependent upon how quickly ATPase of myosins hydrolyzes ATP to produce cross-bridge action. Fast fibers hydrolyze ATP approximately twice as quickly as slow fibers, resulting in much quicker cross-bridge cycling (which pulls the thin filaments toward the center of the sarcomeres at a faster rate). The primary metabolic pathway used by a muscle fiber determines whether the fiber is classified as oxidative or glycolytic. If a fiber primarily produces ATP through aerobic pathways, it is oxidative. More ATP can be produced during each metabolic cycle, making the fiber more resistant to fatigue. Glycolytic fibers primarily create ATP through anaerobic glycolysis, which produces less ATP per cycle. As a result, glycolytic fibers fatigue at a quicker rate.

Inflammation and trauma to a joint (synovial membrane & cartilage), or to tendons or ligaments surrounding the joint may present with a similar clinical presentations. This makes differential diagnosis difficult, particularly by a paramedic or less clinically expert person. Though EMG is used in the diagnosis of neuromuscular conditions, in its current usage it is unsuitable for differentiating joint vs muscular lesions.

In summary, known EMG-based techniques for assessing neuromuscular pathology are poor at providing differential diagnosis. Furthermore, reliable EMG analysis relies on invasive needle-based approached which cause discomfort to the subject. An improved EMG-based approach able to overcome one or more of the above problems would be of advantage.

## SUMMARY OF THE INVENTION

The invention is defined by the claims.

According to examples in accordance with an aspect of the invention, there is provided A processing arrangement for use in mapping anatomical locations of a plurality of muscle fibers belonging to a same motor unit (MU) of a patient, comprising:

> an input/output adapted to communicatively couple with a control input of a TENS stimulation device, and to communicatively couple with a signal output of a surface electromyography (sEMG) sensing arrangement, the sEMG sensing arrangement comprising a plurality of sEMG sensors for placement during use on the skin around a muscle of interest in a pre-defined geometrical arrangement; and one or more processors adapted to perform a localization operation comprising:

> controlling a power and/or frequency setting of the TENS stimulation device to stimulate activation of muscle fibers of a target motor unit of a muscle of interest;

>> receiving a respective sEMG sensor readout signal from each of the plurality sEMG sensors over a sampling period; identifying a series of signal spikes in each of the sensor readout signals, each spike associated with an action potential of a respective one of a plurality of muscle fibers belonging to the target motor unit; determining for each signal spike of each sEMG sensor readout signal, an estimate of a distance, d, of the sEMG sensor from the associated muscle fiber from which the spike originates, based on time-of-arrival of the respective signal spike;

> determining locations of the plurality of muscle fibers relative to the sEMG sensors based on the known geometrical arrangement of the sEMG sensors.

Embodiments of the proposed invention are based on identifying locations of individual muscle fibers within individual motor units. This is achieved based on controlled use of TENS to stimulate recruitment of individual motor units, and use of triangulation to detect individual muscle fiber locations. This allows for more precise differential diagnosis, as well as a variety of clinically relevant information arising from mapping of muscle fiber positions.

When stimulated or recruited, muscle fibers exhibit rapid twitching behavior which results in emission of a pulsing electrical signal with a certain pulse frequency. The pulse period of a single fiber will typically be larger than the time-of-arrival difference at a single sEMG sensor of the pulses from the plurality of muscle fibers. Thus, in practice each sEMG sensor will record a series of electrical spikes from the plurality of fibers which periodically repeats. The localization calculation is performed for just a single instance of this series of spikes.

Crucially, the method relies on the additional use of TENS to implement controlled recruitment of individual motor units. Rather than relying on a subject to contract a muscle, in which event typically multiple motor units are recruited, use of TENS allows more precise control over motor unit stimulation. This ensures that when signal spikes are received at a respective sEMG sensor, it can be known with certainty that the spikes all correspond to muscle fibers of a single target motor unit only. This is important since muscle fibers of a single motor unit will all fire simultaneously, which thus enables localization of individual muscle fibers to be performed using a triangulation operation (wherein time of arrival difference between respective signal spikes can be assumed to be associated with differences in the distance of the signal source only, without any difference in the signal activation time). Thus, embodiments of the present invention avoid complications of previous sEMG sensing methods wherein decomposition of action potential trains of multiple motor units must be performed.

The power and/or frequency level at which to set the TENS to stimulate a target motor unit may be pre-determined in advance, or it can be identified in real time through a process of graduated incrementing of TENS power until a motor unit stimulation is achieved. At this point, power incrementing can be paused and sensor data collected. This can be repeated multiple times to acquire data for each of a series of motor units and map the positions of muscle fibers for each motor unit. At each recruitment of a new motor unit, although the previous motor unit is also activated, the newly recruited motor unit appears as an additional set of spikes superposed on the previous signal. The previous signal can simply be subtracted from the new signal, allowing the muscle fiber localization process for the new motor unit to be performed using just the newly detected set of spikes.

Thus, to localize fibers for a series of motor units, the method can comprise the one or more processors transitioning the TENS stimulation device through a series of TENS power and/or frequency settings, to successively recruit each of a series of different target motor units of the muscle of interest.

Determining the locations of the plurality of muscle fibers may further be based on an estimated signal speed-of-travel through the tissue between the muscle fiber and the skin surface. This may be a pre-determined value used in the calculations.

In some embodiments, the method may comprise determining a respective arc or spherical shell of candidate positions for each muscle fiber of radius d about each respective sEMG sensor. In some embodiments, the method further comprises, based on the known geometrical arrangement of the sEMG sensors, determining locations of the plurality of muscle fibers relative to the sEMG sensors based on identifying points of intersection of the determined arcs or spherical shells for the plurality of sEMG sensors.

Here, the method effectively define a multi-source triangulation operation. The muscle fibers are treated as effective point sources. For a single source triangulation, a minimum of three sensors would be needed. For a multi-source triangulation, preferably, the number of sEMG sensors exceeds three by the same number as the number of additional sources over one. For each sEMG sensor, there will be a plurality of arcs or shells corresponding to candidate positions for each one of the muscle fibers. The spatial points at which a respective arc or spherical shell from all of the sEMG sensors overlap correspond to locations of single muscle fibers. By identifying all of these locations of universal overlap, the locations of all of the muscle fibers can be determined.

The localization operation further may comprise deriving, based on a position of the sEMG sensor arrangement relative to the patient anatomy, a map of anatomical locations of the muscle fibers of the respective motor unit within the patient.

The map may comprise a set of co-ordinate positions of the muscle fibers (treated as point sources across a cross-sectional plane of the muscle) within the patient anatomy.

The map may be referred to as an activity map. An activity map represents muscle fiber (electrical) activity. The map may also be referred to as a functional image of the muscle fiber activity.

Determining the estimate of the distance, d, of an associated muscle fiber from a given sEMG sensor may be further based on an amplitude of the spike, and based on an estimate of a signal attenuation as function of distance through the tissue between the muscle and the skin surface.

The controlling the TENS power and/or frequency setting may comprise: gradually increasing a TENS power and/or frequency level from zero, and simultaneously monitoring the sEMG sensor readout signals; and stopping the increase in TENS power and/or frequency level responsive to detecting a signal spike of a threshold amplitude in at least one of the sEMG sensor readout signals.

Here, the required TENS power and/oror frequency level for recruiting a first single motor unit is dynamically determined in real time based on smoothly or incrementally increasing the TENS power until muscle fibers of the motor unit first begin to activate, this being determined based on detecting a signal spike in at least one of the sEMG sensors of threshold magnitude.

The setting of the TENS power and/or frequency may further comprise holding the TENS power and/or frequency level steady after stopping the increase.

In the above, the detecting of the threshold signal amplitude may (e.g. automatically) trigger a start the said sampling period. In this case, as soon as the spike of the threshold amplitude is detected (indicating motor unit recruitment), the sampling period referred to previously begins, and determining of the muscle fiber locations performed based on the recorded signal spikes in the sampling period.

The localization operation may be repeated for a plurality of different TENS stimulation power and/or frequency levels, each new power and/or frequency level for stimulating muscle fibers of a further respective motor unit, for thereby determining muscle fiber locations for a plurality of different respective motor units.

Transitioning the TENS power between the plurality of different TENS power and/or frequency levels may comprise, following completion of a sampling period for one TENS power and/or frequency level, gradually increasing the TENS power until a new signal spike of a threshold amplitude is detected in at least one of the sEMG sensor readout signals, and holding the power and/or frequency level steady responsive to said detection.

A next sampling period may begin (e.g. automatically) responsive to the detection of said signal spike of a threshold amplitude.

According to one or more embodiments, the processing arrangement may be operable in a further operation mode for use with the plurality of sEMG sensors placed covering an agonist/antagonist muscle pair. The further operation mode may comprise: receiving from sEMG sensors covering the agonist one or more sEMG sensor readings for the agonist at rest;

receiving from sEMG sensors covering the antagonist one or more sEMG sensor readings for the antagonist at rest;

comparing the agonist and antagonist sensor readings with an agonist and antagonist reference level respectively; and

comparing the agonist and antagonist sensor readings with one another.

In the case of the agonist and antagonist sensor readings differing from one another by more than a pre-defined first threshold, and one or both of the agonist and antagonist differing from the respective reference level by a pre-defined further threshold, the further operation mode may comprise generating an information output indicative of presence of a muscle, tendon or ligament pathology.

In the case of the agonist and antagonist sensor readings not differing from one another by more than the pre-defined first threshold, and one or both of the agonist and antagonist differing from the respective reference level by a pre-defined further threshold, the further operation mode may comprise generating an information output indicative of presence of a joint pathology.

This operation mode aims to differentially distinguish different types of pathology based on comparing sEMG sensor readings for each of an agonist and antagonist pair. If at least one of the two muscles exhibits an unusually high sEMG reading when at rest, this may indicate tension, which may indicate a pathology. The defined operation mode aims to determine whether this is more likely caused by a muscle or a joint pathology. If the readings of the agonist and antagonist are relatively similar, this would indicate that the pathology is originating at the level of the joint, and so affecting both muscles of the pair roughly evenly. If the readings of the agonist and antagonist are asymmetric, this may indicate that the pathology is originating more at the level of the individual muscle.

In accordance with one or more embodiments, the processing arrangement may be operable in a further operation mode wherein the input/output is adapted to further communicatively couple with a force sensor for sensing a contraction force of the muscle of interest, and the further operation mode comprising:

a first measurement phase, for execution after contraction of the muscle by the subject for a first time duration, the first measurement phase comprising recording first sEMG sensor data from the sEMG sensors, and simultaneously recording a force signal, F1, from the force sensor; and

a second measurement phase for execution after contraction of the muscle by the subject for a second time duration, longer than the first, the second measurement phase comprising recording second sEMG sensor data from the sEMG sensors, and simultaneously recording a force signal, F2, from the force sensor.

This further operation mode may further comprise applying both a first and second filter to each of the first and second sEMG sensor data to derive relative higher frequency and relative lower frequency signal components of each of the first and second sEMG sensor data.

This further operation mode may comprise determining a ratio of fast-twitch to slow-twitch muscle fibers in the muscle of interest based on the equation

$$F2 = F1 \times \frac{100 - x}{xr} \times S$$

where x is the percentage of fast muscle fibers in the muscle of interest, r is the ratio of the force generated by the same number of fast muscle fibers and slow muscle fibers, and S is given by the ratio of the low frequency first sEMG signal data to the low frequency second sEMG data.

The terms higher and lower in this context mean higher and lower relative to one another.

The first filter mentioned above may be a bandpass filter with a frequency band having a lower frequency bound between 120 Hz and 130 Hz and an upper frequency bound between 240 Hz and 260 Hz. The second filter may be a low pass filter having an upper frequency bound of between 110 Hz and 130 Hz.

In each of the first and second measurement phases, the recording of the sEMG sensor data may be performed conditionally upon a rate of change of the force signal with respect to time being below a pre-defined threshold.

The threshold may be at or close to zero.

This ensures that the muscle activation is stable, and is not in a transient stage in which additional motor units are being recruited.

Examples in accordance with a further aspect of the invention provide a system comprising:

> an sEMG sensing arrangement, the sEMG sensing arrangement comprising a plurality of sEMG sensors for placement during use on the skin of a subject around a muscle of interest in a pre-defined geometrical arrangement;
> a TENS stimulation device including electrodes for use in stimulating the muscle of interest; and
> a processing arrangement in accordance with any example or embodiment outlined above or described below, or in accordance with any claim of this application, the input/output of the processing arrangement communicatively coupled with the sEMG sensing arrangement and the TENS stimulation device.

Examples in accordance with a further aspect of the invention provide A method for use in mapping anatomical locations of a plurality of muscle fibers belonging to a same motor unit (MU) of a patient, the method comprising controlling:

> an sEMG sensing arrangement, the sEMG sensing arrangement comprising a plurality of sEMG sensors placed on the skin of the subject around a muscle of interest in a pre-defined geometrical arrangement, and
> a TENS stimulation device comprising electrodes arranged for stimulating the muscle of interest;

the method comprising performing a localization operation comprising:

> controlling a power or frequency setting of the TENS stimulation device to stimulate activation of muscle fibers of a motor unit of a muscle of interest;
>
> > receiving a respective sEMG sensor readout signal from each of the plurality sEMG sensors over a sampling period;
> > identifying a series of signal spikes in each of the sensor readout signals, each spike associated with an action potential of a respective one of the plurality of muscle fibers of the motor unit;
> > determining for each signal spike of each sEMG sensor readout signal, an estimate of a distance, d, of the associated muscle fiber from which the spike originates;
>
> determining locations of the plurality of muscle fibers relative to the sEMG sensors based on the known geometrical arrangement of the sEMG sensors.

The method may be at least partly computer implemented. For example, at least the localization operation maybe computer implemented.

In some examples, the method may further comprise a preliminary step of placing the sEMG sensors on the body in the defined geometrical arrangement around the muscle of interest.

This step may in some examples be performed manually by a user or operator, while the localization operation may in some examples be performed automatically by a processor or computer.

Examples in accordance with a further aspect of the invention provide a computer program product comprising computer program code, the computer program code being executable on a processor, wherein, when the processor is operatively coupled with an sEMG sensor arrangement, and a TENS stimulation device, the code configured to cause the processor to perform a method in accordance with any example or embodiment outlined above or described below, or in accordance with any claim of this application.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

> Fig. 1 shows an example system comprising a processing arrangement according to one or more embodiments;
> Fig. 2 illustrates localization of a muscle fiber;
> Fig. 3 illustrates example EMG signals measured at a set of sEMG electrodes;
> Fig. 4 illustrates triangulation of multiple muscle fiber locations;
> Fig. 5 outlines a workflow for performing an example method for localizing muscle fibers;
> Fig. 6 outlines an example processing flow for differentially identifying pathologies; and
> Fig. 7 outlines an example workflow for analyzing fast-twitch and slow-twitch muscle fiber composition of a muscle.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a means for mapping locations of individual muscle fibers of each of one or more single motor units based on use of TENS for controlled recruitment of motor units, and based on an array of sEMG electrodes placed around the relevant anatomical location. Activation of individual muscle fibers can be detected as individual spikes in the signal at each respective sEMG electrode. The time of arrival of each spike in each sEMG electrode signal can be used to estimate a radial distance of the corresponding muscle fiber from the electrode. From this, triangulation of the locations of each muscle fiber can be performed.

A plurality of non-invasive surface EMG sensors may be used. These may be standalone sensor elements, or a sensor unit may be provided comprising a substrate or housing to which are mounted an arrangement of multiple sEMG sensors. For example, a sensor unit may be provided in form of a patch comprising multiple sEMG sensors integrated therein. An individual sEMG sensor may mean an sEMG electrode for example. The sensor unit may in some examples be integrated additionally with an inertial measurement unit (IMU) or force sensor for sensing muscle power.

A number of different embodiments of the invention are provided, features of any one of which may be combined with any other embodiment.

In one or more sets of embodiments, time of arrival difference of signals at each of a plurality of EMG sensors is used to identify individual muscle fibers.

In one or more embodiments, signals may be received from agnostic and antagonist muscles to determine a location or source of a pathology. For example, differential determination may be performed between (i) a muscle, tendon or ligament pathology, and (ii) a joint pathology.

In one or more embodiments, analysis may be performed regarding composition of a muscle fiber between different muscle fiber types: Type 1 (slow twitch, e.g. slow oxidative), and Type 2 (Fast twitch). This may be based on spectral analysis, e.g. application of a Fourier (or related) transform of the EMG signal. Slow fiber activity may be identified through filtering the EMG signal using a low pass filter (e.g. <125Hz). Fast fiber activity may be identified using a band pass filter (126-250Hz). This measurement may be combined with force measurement (e.g. using a dynamometer).

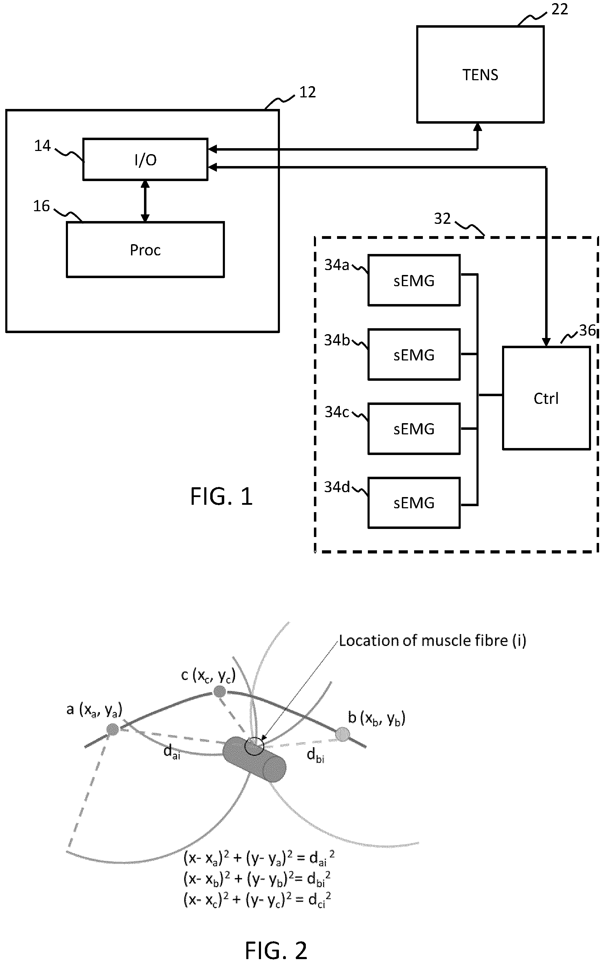

Fig. 1 shows components of an example system in accordance with one or more embodiments of the invention. The system comprises a processing arrangement 12. The processing arrangement 12 is for use in determining anatomical locations of a muscle fibers belonging to one or more target motor units (MU) of a patient. The processing arrangement 12 comprises an input/output (I/O) 14 adapted to communicatively couple with a control input of a TENS stimulation device 22, and to communicatively couple with a signal output of a surface electromyography (sEMG) sensing arrangement 32.

The sEMG sensing arrangement 32 comprises a plurality of sEMG sensors 34a-34d for placement during use on the skin around a muscle of interest in a predefined geometrical arrangement. In the illustrated example, the sEMG sensing arrangement in an sEMG sensing apparatus comprising a controller 36 which is operatively coupled with each of the sEMG sensors. The I/O 14 of the processing arrangement 12 is communicatively coupled with the controller 36 of the sEMG sensing apparatus in this example. In other examples, the processing arrangement may be arranged to connect directly to the sEMG sensor elements 34. In either case, the I/O receives a respective sEMG sensor signal detected by each sEMG sensor 34.

The processing arrangement 12 further comprises one or more processors 16 operatively coupled with the I/O 14. The one or more processors are adapted to perform a localization operation.

The localization operation comprises controlling the TENS stimulation device to set a TENS stimulation power and/or frequency level to stimulate simultaneous activation of muscle fibers belonging to a target motor unit. The power or frequency level may be predetermined, or may be determined in real time as will be described below.

The localization operation further comprises receiving a respective sEMG sensor readout signal from each of the plurality sEMG sensors over a sampling period.

The localization operation further comprises identifying a series of signal spikes in each of the sensor readout signals, each spike associated with an action potential of a respective one of the set of muscle fibers of the motor unit.

The localization operation further comprises determining locations of the plurality of muscle fibers based on a time-of-arrival of each respective signal spike in each respective sEMG sensor readout signal.

For example, for each spike, an estimate of a radial distance, d, between the respective sEMG sensor and the associated muscle fiber from which the spike originates may be determined.

If the relative positions of the sensors is known, the locations of the muscle fibers may be determined based on identifying crossing points of respective radial arcs from each sensor of radius $d_{ni}$, where $n$ indexes the sensor and $i$ indexes the spike (or muscle fiber).

One aspect of the invention provides the processing arrangement 12 alone. A further aspect of the invention provides a system comprising the processing arrangement and the TENS stimulation device 22 and/or the sEMG sensing arrangement 32.

Although only four sEMG sensors are shown in Fig. 1, any number of sEMG sensors may be provided in other examples.

In use, the non-invasive sEMG sensors 34a-34d would be placed across an area of the surface of the affected anatomy, so that each muscle or muscle group around the relevant joint is covered by the area. As the sensors are non-invasive, any number of sEMG sensors can be provided without causing discomfort to the patient. Therefore a larger number can be used than in typical invasive (needle electrode) sEMG sensing.

As will be explained below, in addition to detecting signals with the muscles at rest, EMG measurement can be performed during movement as well. A common issue with state of the art EMG measurement is that the small number of needle electrodes are insufficient to record and/or discriminate between activations of all motor units of a muscle. There is therefore often a mismatch between mechanical effort and electrical activity recorded through EMG, even after accounting for the type of contraction (isotonic vs isometric) or neuromuscular pathologies. Since a plurality of electrodes may be used in the proposed embodiments, activity of an individual muscle fiber (belonging to a motor unit) can be isolated based on a predicted intensity loss (attenuation) and time delay dependent on the distance of the sensor from the muscle fiber. As all the fibers belonging to a same motor unit will fire simultaneously, by identifying and locating these fibers within the muscle, the motor units may be mapped.

In use, embodiments employ TENS for controlled recruitment of motor units. Voluntary contraction of a muscle by a subject can typically recruit multiple motor units, particularly if the subject is unable to modulate force sufficiently slowly. To ensure that not all motor units are recruited simultaneously, transcutaneous electrical nerve stimulation (TENS) can be employed. The stimulation may be varied gradually to ensure slow successive recruitment of motor units.

The principles behind localization of muscle fibers in accordance with embodiments of the present invention will now be outlined in more detail.

In a first step, the processing arrangement 12 may be adapted to apply TENS stimulation at a first TENS stimulation power, to achieve recruitment of a first single motor unit. The multiple muscle fibers belonging to the single motor unit will fire simultaneously. The signals are in phase. Each of the plurality of EMG sensors at the skin surface will record an EMG readout signal comprised of a series of spikes, each spike corresponding to an action potential of a respective one of the muscle fibers of the motor unit.

By way of example, and without loss of generality, an example may be considered of an array of sEMG sensors positioned at the skin surface around a muscle of interest. The array may be annular, or may be a different configuration. Locations of individual muscle fibers within a cross section of the muscle under investigation are to be determined in this example.

In general, the intensity of a signal at an sEMG sensor received from a single muscle fiber may be approximated by a power function $(j)=k \cdot (r(j)/r_k)^{-Q}$, where $r(j)$ is the radial distance of the sensor to the muscle fiber, $V(j)$ is the surface amplitude, $k$ is a constant representing estimated amplitude for the signal at distance $r_k$, and $Q$ is a constant representing the strength of signal attenuation. The constants may be pre-determined, for instance based on historical data for prior subjects.

The measurable surface signal should be strongest at the point with the shortest radial distance, which is directly above the muscle fiber. For this point, the amplitude is $V(j) = V_{max}$ and distance is $r(j) = d$, where d the depth of the fiber. The position on the surface where the measurable signal reaches half maximum may be represented as $V_F$ with radial distance to the muscle fiber of $r_F$. This point may be identified based on determining a distribution of the sEMG signal across the skin using the sensor readout signals of the plurality of sEMG sensors. By combining the equation for $V_{max}$ and $V_F$, the following general equations can be derived:

$$V_{max} = k\left(\frac{d}{r_k}\right)^{-Q}, V_F = k \cdot \left(\frac{r_F}{r_k}\right)^{-Q}$$

$$\Rightarrow \frac{V_{max}}{V_F} = \left(\frac{d}{r_F}\right)^{-Q} \Rightarrow 2 = \left(\frac{r_F}{d}\right)^{Q}$$

$$\Rightarrow \frac{r_F}{d} = 2^{1/Q}$$

This shows that the signal intensity (due to activation of a single muscle fiber) at a single sEMG sensor depends solely on the sensor-fiber distance.

Phase of the signal could also be taken into account since the signal will reach the sensor with a delay proportional to the sensor-fiber distance.

Since both the signal intensity and the signal phase are functions of sensor-fiber distance, it is possible to triangulate the location of a single fiber based on sEMG sensor readings from multiple sEMG sensors.

For example, Fig. 2 illustrates a simplified triangulation scenario in which a single muscle fiber activates, and three sEMG sensors (a, b, c) are positioned around the muscle fiber. The (x, y) co-ordinates of each sEMG sensor are shown.

For this triangulation scenario, a cross-section through the muscle is considered. The signal at time $t$ at an sEMG sensor $j$ due to a muscle fiber $i$ (where the muscle fiber is treated as a point source) can be repre-

$$Ae^{-\frac{d_i}{D}}\delta\left(t - \tau(d_{ji})\right),$$

sented by where $d_{ji}$ is the distance of the muscle fiber $i$ from the sEMG sensor $j$, A and D are constants, $\delta$ is the impulse function and $\tau$ is the

time of arrival at the sEMG sensor of the signal spike corresponding to the muscle fiber firing (action potential). This is a function of distance $d_{ji}$.

From the point of view of an individual sEMG sensor, the muscle fiber would be expected to lie anywhere on an arc or circle or sphere of radius $d_{ji}$ centered on the sEMG sensor. Identifying the point of intersection of the respective arcs, circles or spheres of the three sEMG sensors a, b, c provides the location of the muscle fiber. Algebraically, this amounts to solving the set of equations:

$$(x - x_a)^2 + (y - y_a)^2 = d_{ai}{}^2$$

$$(x - x_b)^2 + (y - y_b)^2 = d_{bi}{}^2$$

$$(x - x_c)^2 + (y - y_c)^2 = d_{ci}{}^2$$

Fig. 2 represents a simplified scenario in which there is only a single muscle fiber to be detected. In reality, when recruiting a motor unit, the plurality of muscle fibers belonging to the motor unit fire simultaneously. Thus, in practice, identifying the location of a single muscle fiber amounts to a multisource triangulation problem. Each sEMG sensor will detect a series of signal spikes corresponding to action potentials of each of the plurality of respective muscle fibers of the motor unit. To identify locations of individual muscle fibers, time of arrival of each spike in each sEMG sensor readout signal may be taken into account.

In this scenario, the composite signal received at respective sEMG sensor may be represented by

$$\sum_{i=0}^{n} A e^{-\frac{d_i}{D}} \delta\left(t - \tau(d_{ji})\right),$$ where $d_{ji}$ is the distance of muscle fiber $i$ from the sEMG sensor $j$, A and D are constants, $\delta$ is the impulse function and $\tau$ is the time of arrival at the sEMG sensor of the signal spike corresponding to the action potential of muscle fiber $i$.

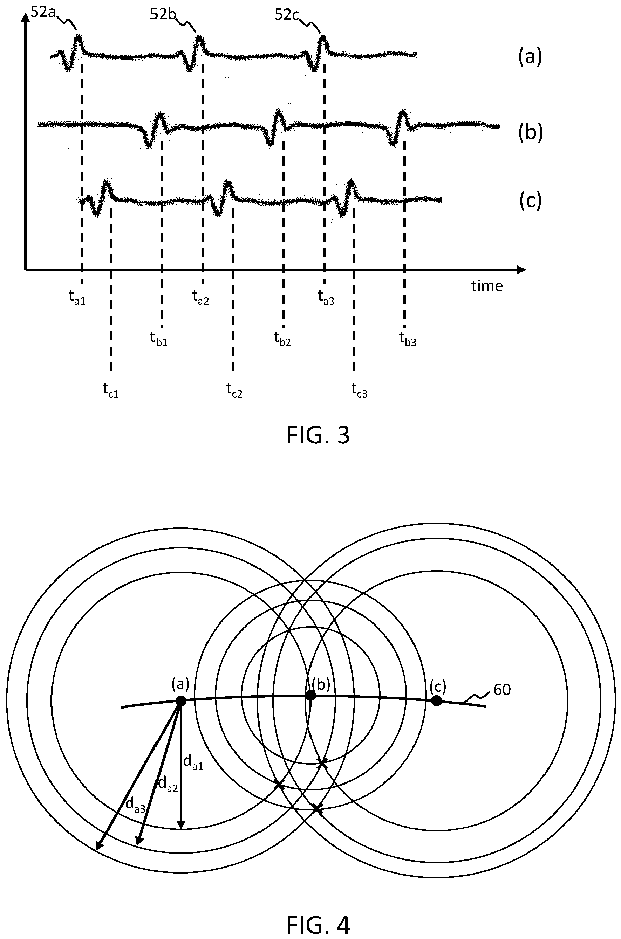

This is represented schematically in Fig. 3, which illustrates a set of example sEMG sensor readout signals from a set of three example sEMG sensors, labelled (a), (b) and (c), each covering a same sampling time period. Each of the sensor readout signals comprises a series of signal spikes 52, each spike associated with an action potential of a respective one of the set of muscle fibers of the motor unit. Each spike has an associated time-of-arrival, $t_{ji}$ as labelled in Fig. 3, where $j$ indexes the sEMG sensor and $i$ indexes the muscle fibers.

The processing arrangement 12 may be adapted to determine for each signal spike 52 of each sEMG sensor readout signal, an estimate of a distance, $d_{ji}$, of the associated muscle fiber, $i$, from which the spike originates to the sEMG sensor $j$, based on the time-of-arrival of the respective signal spike.

The processing arrangement 12 may be further adapted to determine for each sEMG sensor, $j$, a respective arc or spherical shell of candidate positions for each muscle fiber $i$, of radius $d_{ji}$ about the respective sEMG sensor.

A geometrical arrangement of the sEMG sensors may be known. The processing arrangement 12 may be adapted to determine locations of the plurality of muscle fibers relative to the sEMG sensors based on identifying points of intersection of the determined arcs or spherical shells for the plurality of sEMG sensors.

This is illustrated schematically in Fig. 4, which shows a set of three example sEMG sensors (a), (b), (c) positioned on a skin surface 60. Three example muscle fiber locations are schematically illustrated with respective crosses in Fig. 4. Based on the acquired sensor readout signals at each sEMG sensor, a set of three arcs or circles or shells are determined surrounding the sEMG sensor location, each of radius $d_{ji}$ about the sEMG sensor location, where $j$ represents the respective sEMG sensor, and $i$ represents the respective muscle fiber.

As illustrated in Fig. 4, points of mutual intersection between respective arcs or shells of all of the sEMG sensors can be identified as locations of muscle fibers (indicated by crosses in Fig. 4).

The determining of the estimate of the distance, $d_{ji}$, of an associated muscle fiber $i$ from which each respective signal spike originates from the sEMG sensor $j$ may in some examples be based on an amplitude of the spike, and based on an estimate of a signal attenuation as a function of distance through the tissue between the muscle and the skin surface.

In some examples, it may occur that more than one muscle fiber is located on the same arc or circle or sphere around a respective sEMG sensor. This case can be identified by detecting the signal intensity of a spike at the sEMG sensor being an integer multiple of the expected signal intensity due to a single muscle fiber.

In some examples, the localization operation may further comprise deriving, based on a position of the sEMG sensor arrangement relative to the patient anatomy, a map of anatomical locations of the muscle fibers of the respective motor unit within the patient. The map may comprise a set of co-ordinate positions of the muscle fibers (treated as point sources across a cross-sectional plane of the muscle) within the patient anatomy. A patient body co-ordinate system may be defined relative to which the co-ordinate positions are defined. Positions of the sEMG sensors relative to this co-ordinate system may be known - e.g. pre-defined in advance, or input by a user. In this way the muscle fiber co-ordinate positions can be determined. The map may comprise in some examples a visual representation of the muscle fiber positions.

The localization operation described above was illustrated for a 2D measurement case, in which the muscle fibers were modeled as point sources in a 2D cross-sectional plane. This approach can be expected to work well for most practical scenarios. However, a three dimensional localization can also be performed. In this case, each muscle fiber may be modeled as a set of collinear points, which are treated as being excited simultaneously, and wherein the arcs are replaced with spherical shells. Instead of being treated as excited simultaneously, in some examples, the collinear points of each muscle fiber may be treated as being excited in series, for example starting from neuromuscular junction end plates.

As explained above, preferably a greater number of sEMG sensors are used than the number of muscle fibers to be detected. However, this is not essential. In cases where there are fewer sEMG sensors than muscle fibers to be detected (an underdetermined system), the localization operation may comprise localizing clusters of neighboring muscle fibers in lieu of individual fibers.

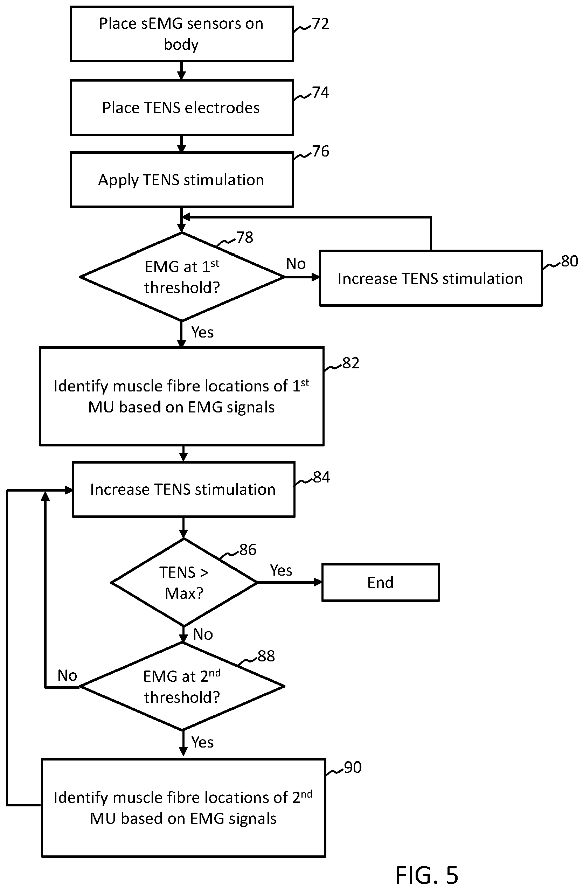

In some examples, the localization operation described above may be repeated for a plurality of motor units. An example method for achieving this will now be described with reference to Fig. 5. Fig. 5 outlines steps of an example workflow for localizing muscle fibers of a plurality of motor units.

Execution of the method may begin by the subject or a clinician placing 72 a plurality of sEMG sensors on the body above or around the muscle or muscle group of interest. The method may further comprise placing 74 electrodes of the TENS stimulation apparatus onto the subject in a position suitable for stimulating one or more motor units of interest.

The method then comprises controlling 76 the TENS stimulation device to set a TENS stimulation power and/or frequency to a first power and/or frequency level to stimulate simultaneous activation of muscle fibers belonging to a single first motor unit (MU).

In some examples, the first power and/or frequency level may be predefined. For example, it may be determined in advance what TENS power and/or frequency level will stimulate the first motor unit and this level may be stored in a local memory of the processing arrangement.

In further examples, setting of the TENS stimulation power to the first power and/or frequency level may comprise: gradually increasing the TENS power and/or frequency level from zero, and simultaneously monitoring the sEMG sensor readout signals; and stopping the increase in TENS power and/or frequency level responsive to detecting a signal spike of a threshold amplitude in at least one of the sEMG sensor readout signals. This approach is shown in Fig. 5 which shows a feedback loop is implemented comprising recurrently probing or sampling the sEMG sensor readout signals, determining 78 whether a first threshold is met, and increasing 80 TENS stimulation if not. Once the first threshold is reached, the TENS power and/or frequency level may be held steady after stopping the increase.

Once the first threshold is detected, the sEMG signals from the plurality of sensors are sampled and recorded over a measurement period. This may be triggered automatically following detection of a spike of the threshold amplitude. The recorded signals are used as the inputs for the localization operation described above to determine 82 the locations of the muscle fibers of the first motor unit.

Once the muscle fibers belonging to the first recruited MU have been localized, the TENS stimulation strength may then be gradually increased 84 to recruit a next MU. Transitioning the TENS power between the plurality of different TENS power and/or frequency levels may comprise, following completion of a sampling period for one TENS power and/or frequency level, gradually increasing 84 the TENS power until a new signal spike of a threshold amplitude is detected in at least one of the sEMG sensor readout signals, and holding the power and/or frequency level steady responsive to said detection. As shown in Fig. 5, a feedback loop may be implemented which comprises increasing 84 the TENS stimulation, sampling at least one of the sEMG sensor readout signals, and determining 88 whether the sEMG signal exceeds a second threshold. If not, the TENS stimulation is increased 84. If the threshold is reached, a next sampling period begins in which the sEMG sensor readout signals are recorded and the localization operation repeated 90 to determine locations of individual muscle fibers of the next motor unit.

Optionally, before testing whether the EMG signal is at a second threshold, a further test may be performed 86 to determine whether the TENS yet exceeds a pre-determined maximum level. If so, the method may be ended. The predetermined maximum TENS level may be pre-set to correspond to a level suitable for recruitment of a certain number of different motor units for example.

The steps 84-90 may in some examples be repeated to recruit further motor units. Here, reference to the second threshold in step 88 may correspond to an $n$th threshold, for recruitment of an $n$th motor unit and reference to the second MU in step 90 may correspond to an $n$th MU.

At each recruitment of a new motor unit, the previous motor unit also remains activated. Hence the newly recruited motor unit appears in each sEMG sensor readout signal as an additional set of spikes superposed on the previous signal. The previous signal can for example simply be subtracted from the new signal, allowing the triangulation process for the new motor unit to be performed using just the newly detected set of spikes.

Methods in accordance with embodiments described above provide a greater amount of clinical information than known EMG sensing methods. Localization of individual muscle fiber activity allows analysis of neuropathology at higher resolution. The information derived can be used to assist in identifying and differentiating between different types of neuromuscular pathology. One illustrative example may be for differentiating between trauma to the neurons and muscular trauma. In case of trauma to neurons, activation of one or more motor units would be absent (short of complete nerve rupture). In cases of muscular trauma, no individual motor unit may be completely non-functional, but a region of muscle may show aberrant or no activity.

In the above method, steps 76 to 90 may be performed by a computer. Steps 72 and 74 may be performed by a user or operator.

In accordance with one or more advantageous embodiments, sEMG sensor readings may be used for differentially distinguishing different types of pathology based on comparing sEMG sensor readings for each of an agonist and antagonist pair.

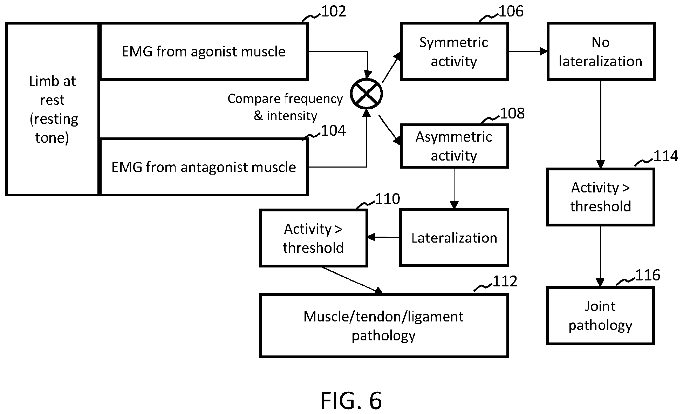

For example, the processing arrangement 12 may comprise at least one operation mode for differentially determining between (i) a muscle, tendon or ligament pathology, and (ii) a joint pathology. The processing workflow for one example of this set of embodiments in shown in Fig. 6.

The patient is asked to hold the relevant body part at rest. The method comprises: receiving 102 from sEMG sensors covering the agonist (e.g. extensor) one or more sEMG sensor readings for the agonist at rest (that is, without movement or movement intention). The method comprises receiving 104 from sEMG sensors covering the antagonist (e.g. flexor) muscle one or more sEMG sensor readings for the antagonist at rest.

The measurement at rest effectively provides a baseline measurement. In the current standard clinical use of EMG, baseline measurements are often ignored. In the case of asymmetric guarding (when one or one group of muscles has a higher tone than the opposing group of muscles), this may occur due to muscular pathology or tendon/ligament injury, or it may occur due to joint pathology. Analysis of the baseline measurements enables detection of differences of muscle tone and possible candidate pathological conditions may be determined with the help of coded clinical knowledge.

The method comprises comparing the agonist and antagonist sensor readings with an agonist and antagonist reference threshold level respectively. For example frequency and intensity of each may be compared with respective reference thresholds for frequency and intensity. This is to determine if there is abnormal resting muscle tone.

The method further comprises comparing the agonist and antagonist sensor readings with one another. For example, the frequencies and intensities of the sEMG measurements from both muscles may be compared.

Two general cases can be distinguished: symmetric activity 106 and asymmetric activity 108. In the case of the agonist and antagonist sensor readings differing from one another by more than a pre-defined first threshold, there is asymmetric activity. This indicates a state of lateralization. In the case of the agonist and antagonist sensor readings not differing from one another by more than the pre-defined first threshold, there is symmetric activity.

In the case of asymmetric activity 108, it is determined 110 whether one or both of the agonist and antagonist differ from the respective reference level (mentioned above) by a pre-defined further threshold. If so, the further operation mode may comprise generating an information output 112 indicative of presence of a muscle, tendon or ligament pathology.

In the case of symmetric activity 106 (no lateralization), it is determined 114 whether one or both of the agonist and antagonist differ from the respective reference level (mentioned above) by a pre-defined further threshold, i.e. observation of an abnormal (increased) tone. If so, the further operation mode may comprise generating an information output 116 indicative of presence of a joint pathology.

The above procedure may be provided as an additional selectable operation mode further to the localization operation described above. Alternatively, one or more embodiments provide a processing arrangement operable to perform this operation mode alone, or without the localization operation.

In accordance with at least one set of embodiments, the processing arrangement 12 may be operable in at least one operation mode to determine information related to muscle fiber type composition of a subject's muscle. For example, a ratio of Type 1 vs Type 2 fibers may be determined. This may be determined based on frequency decomposition of the obtained sEMG signals. It may be further based on force data pertaining to the muscle.

By way of example, the input/output 14 may be adapted to further communicatively couple with a force sensor (e.g. a dynamometer) for sensing a contraction force of the muscle of interest.

The measurement process may comprise a first 130a and second 130b measurement phase. The first measurement phase 130a, is for execution after contraction 142 of the muscle by the subject for a first time duration. The first measurement phase comprises recording 144 first sEMG sensor data from the sEMG sensors, and simultaneously recording 146 a force signal, F1, from the force sensor.

The second measurement phase 130b is for execution after contraction 152 of the muscle by the subject for a second time duration, longer than the first. The second measurement phase comprises recording 154 second sEMG sensor data from the sEMG sensors, and simultaneously recording 156 a force signal, F2, from the force sensor.

The method comprises extracting 150, 160 high and low frequency components of the first and second sEMG sensor data respectively. This may be achieved by applying both a first and second filter to each of the first and second sEMG sensor data to derive relative higher frequency and relative lower frequency signal components of each of the first and second sEMG sensor data.

The method may further comprise determining 162 a ratio of fast-twitch to slow-twitch muscle fibers in the muscle of interest based on the equation

$$F2 = F1 \times \frac{100 - x}{xr} \times S$$

where $x$ is the percentage of fast-twitch muscle fibers in the muscle of interest, $r$ is the ratio of the force generated by the same number of fast muscle fibers and slow muscle fibers, and S is given by the ratio of the low frequency first sEMG signal data to the low frequency second sEMG data.

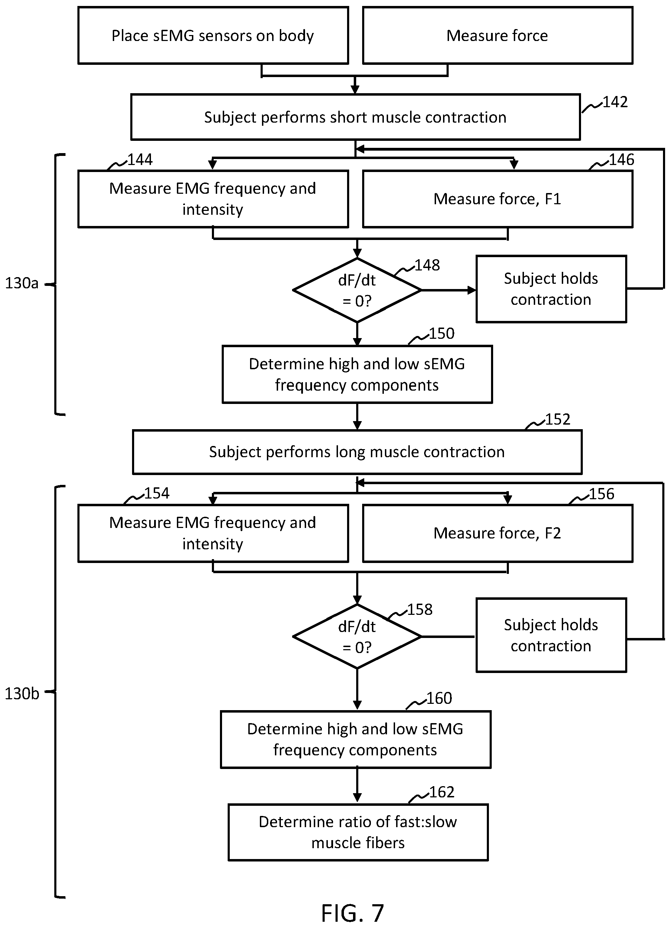

In some examples, and as illustrated in Fig. 7, each of the first 130a and second 130b measurement phases, the recording of the sEMG sensor data and/or the determining of the high and low frequency components is performed conditional upon a rate of change of the force signal with respect to time 148, 158 being below a pre-defined threshold. Preferably the threshold may be at or close to zero. If the said rate of change is not below the pre-defined threshold, the subject may be asked to hold the contraction for longer. This avoids taking measurements during a transient stage (indicated by a nonzero derivative, which would indicate ongoing recruitment of new motor units).

In particular examples, the first filter may be a band-pass filter with a frequency band having a lower frequency bound between 120 Hz and 130 Hz (e.g. 126 Hz) and an upper frequency bound between 240 Hz and 260 Hz (e.g. 250 Hz). The second filter may be a low pass filter having an upper frequency bound of between 110 Hz and 130 Hz (e.g. 125 Hz).

Fast-twitch fibers get fatigued during a sustained load. Following the longer muscle contraction 152 therefore, there will be measurable reduction in the generated force F2 compared to the force F 1 after the short muscle contraction 142. The longer muscle contraction may for example be approximately 2 minutes. The shorter muscle contraction may be for example 10-15 seconds. The shorter contraction may be a muscle contraction against a heavy load.

Determining a ratio of fast to slow muscle fibers may assist in determining a seat of a pathology, for example in case one particular muscle of the same muscle group or in the same anatomical location is diseased/injured. By determining muscle fiber composition, the particular muscle can be identified (e.g. soleus vs gastrocnemius, which differ in their type 1/type 2 fiber ratio).

In accordance with one or more embodiments, a sensor unit may be provided which comprises the plurality of sEMG sensors and further integrates a force sensor for sensing contraction of a muscle.

Embodiments of the invention described above employ a processing arrangement. The processing arrangement may in general comprise a single processor or a plurality of processors. It may be located in a single containing device, structure or unit, or it may be distributed between a plurality of different devices, structures or units. Reference therefore to the processing arrangement being adapted or configured to perform a particular step or task may correspond to that step or task being performed by any one or more of a plurality of processing components, either alone or in combination. The skilled person will understand how such a distributed processing arrangement can be implemented. The processing arrangement includes a communication module or input/output for receiving data and outputting data to further components.

The one or more processors of the processing arrangement can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A processing arrangement for use in mapping anatomical locations of a plurality of muscle fibers belonging to a same motor unit (MU) of a patient, comprising:

   an input/output adapted to communicatively couple with a control input of a TENS stimulation device, and to communicatively couple with a signal output of a surface electromyography (sEMG) sensing arrangement, the sEMG sensing arrangement comprising a plurality of sEMG sensors for placement during use on the skin around a muscle of interest in a pre-defined geometrical arrangement; and
   one or more processors adapted to perform a localization operation comprising:

      controlling a power and/or frequency setting of the TENS stimulation device to stimulate activation of muscle fibers of a target motor unit of the muscle of interest;

      receiving a respective sEMG sensor readout signal from each of the plurality sEMG sensors over a sampling period;
      identifying a series of signal spikes in each of the sensor readout signals, each spike associated with an action potential of a respective one of a plurality of muscle fibers belonging to the target motor unit;
      determining for each signal spike of each sEMG sensor readout signal, an estimate of a distance, d, of the sEMG sensor from the associated muscle fiber from which the spike originates, based on time-of-arrival of the respective signal spike;

      determining locations of the plurality of muscle fibers relative to the sEMG sensors based on the known geometrical arrangement of the sEMG sensors.

2. A processing arrangement as claimed in claim 1, wherein the one or more processors are adapted to transition the TENS stimulation device through a series of TENS power and/or frequency settings, to successively recruit each of a series of different target motor units of the muscle of interest.

3. A processing arrangement as claimed in claim 1 or 2, wherein controlling the TENS power and/or frequency setting comprises:

   gradually increasing a TENS power and/or frequency level from zero, and simultaneously monitoring the sEMG sensor readout signals; and
   stopping the increase in TENS power and/or frequency level responsive to detecting a signal spike of a threshold amplitude in at least one of the sEMG sensor readout signals.

4. A processing arrangement as claimed in claim 3, further comprising holding the TENS power and/or frequency level steady after stopping the increase, and optionally wherein the detecting the threshold signal amplitude triggers a start of said sampling period.

5. A processing arrangement as claimed in any of claims 1-4, wherein the localization operation is repeated for a plurality of different TENS stimulation power and/or frequency levels, each power and/or frequency level for stimulating muscle fibers of a further motor unit corresponding to the muscle of interest, for thereby determining muscle fiber locations for a plurality of different respective motor units.

6. A processing arrangement as claimed in claim 5, wherein transitioning the TENS power between the plurality of different TENS power and/or frequency levels comprises, following completion of a sampling period for one TENS power and/or frequency level, gradually increasing the TENS power and/or frequency until a new signal spike of a threshold amplitude is detected in at least one of the sEMG sensor readout signals, and holding the power and/or frequency level steady responsive to said detection.

7. A processing arrangement as claimed in any of claims 1-6, wherein the localization operation further comprises deriving, based on a position of the sEMG sensor arrangement relative to the patient anatomy, a map of anatomical locations of the muscle fibers of the respective motor unit within the patient.

8. A processing arrangement as claimed in any of any of claims 1-7, wherein the determining the estimate of the distance, d, of an associated muscle fiber from which each respective signal spike originates is further based on an amplitude of the spike, and based on an estimate of a signal attenuation as function of distance through the tissue between the muscle and the skin surface.

9. A processing arrangement as claimed in any of claims 1-8, wherein the processing arrangement is operable in a further operation mode for use with the plurality of sEMG sensors placed covering an agonist/antagonist muscle pair, the further operation mode comprising:

receiving from sEMG sensors covering the agonist one or more sEMG sensor readings for the agonist at rest;
receiving from sEMG sensors covering the antagonist one or more sEMG sensor readings for the antagonist at rest;
comparing the agonist and antagonist sensor readings with an agonist and antagonist reference level respectively;
comparing the agonist and antagonist sensor readings with one another;
in the case of the agonist and antagonist sensor readings differing from one another by more than a pre-defined first threshold, and one or both of the agonist and antagonist differing from the respective reference level by a pre-defined further threshold, generating an information output indicative of presence of a muscle, tendon or ligament pathology; and
in the case of the agonist and antagonist sensor readings not differing from one another by more than the pre-defined first threshold, and one or both of the agonist and antagonist differing from the respective reference level by a pre-defined further threshold, generating an information output indicative of presence of a joint pathology.

10. A processing arrangement as claimed in any of claims 1-9, wherein the processing arrangement is operable in a further operation mode wherein the input/output is adapted to further communicatively couple with a force sensor for sensing a contraction force of the muscle of interest, and the further operation mode comprising:

a first measurement phase, for execution after contraction of the muscle by the subject for a first time duration, the first measurement phase comprising recording first sEMG sensor data from the sEMG sensors, and simultaneously recording a force signal, F1, from the force sensor; and
a second measurement phase for execution after contraction of the muscle by the subject for a second time duration, longer than the first, the second measurement phase comprising recording second sEMG sensor data from the sEMG sensors, and simultaneously recording a force signal, F2, from the force sensor;
applying both a first and second filter to each of the first and second sEMG sensor data to derive relative higher frequency and relative lower frequency signal components of each of the first and second sEMG sensor data;

determining a ratio of fast-twitch to slow-twitch muscle fibers in the muscle of interest based on the equation

$$F2 = F1 \times \frac{100 - x}{xr} \times S$$

where x is the percentage of fast muscle fibers in the muscle of interest, *r* is the ratio of the force generated by the same number of fast muscle fibers and slow muscle fibers, and S is given by the ratio of the low frequency first sEMG signal data to the low frequency second sEMG data.

11. A processing arrangement as claimed in claim 10, wherein the first filter is a band-pass filter with a frequency band having a lower frequency bound between 120 Hz and 130 Hz and an upper frequency bound between 240 Hz and 260 Hz; and
wherein the second filter is a low pass filter having an upper frequency bound of between 110 Hz and 130 Hz.

12. A processing arrangement as claimed in claim 10 or 11, wherein, in each of the first and second measurement phases, the recording of the sEMG sensor data is performed conditional upon a rate of change of the force signal with respect to time being below a pre-defined threshold.

13. A system, comprising:

an sEMG sensing arrangement, the sEMG sensing arrangement comprising a plurality of sEMG sensors for placement during use on the skin of a subject around a muscle of interest in a pre-defined geometrical arrangement;
a TENS stimulation device including electrodes for use in stimulating the muscle of interest; and
a processing arrangement as claimed in any of claims 1-12, the input/output of the processing arrangement communicatively coupled with the sEMG sensing arrangement and the TENS stimulation device.

14. A method for use in mapping anatomical locations of a plurality of muscle fibers belonging to a same motor unit (MU) of a patient,
the method comprising controlling:

an sEMG sensing arrangement, the sEMG sensing arrangement comprising a plurality of sEMG sensors placed on the skin of the subject around a muscle of interest in a pre-defined geometrical arrangement, and
a TENS stimulation device comprising electrodes arranged for stimulating the muscle of interest;

the method comprising performing a localization operation comprising:

controlling a power and/or frequency setting of the TENS stimulation device to stimulate activation of muscle fibers of a target motor unit of the muscle of interest;

receiving a respective sEMG sensor readout signal from each of the plurality sEMG sensors over a sampling period;
identifying a series of signal spikes in each of the sensor readout signals, each spike associated with an action potential of a respective one of a plurality of muscle fibers of the target motor unit;
determining for each signal spike of each sEMG sensor readout signal, an estimate of a distance, d, of the associated muscle fiber from which the spike originates;

determining locations of the plurality of muscle fibers relative to the sEMG sensors based on the known geometrical arrangement of the sEMG sensors.

15. A computer program product comprising computer program code, the computer program code being executable on a processor,
wherein, when the processor is operatively coupled with an sEMG sensor arrangement, and a TENS stimulation device, the code configured to cause the processor to perform a method in accordance with claim 14.

FIG. 1

$$(x- x_a)^2 + (y- y_a)^2 = d_{ai}{}^2$$
$$(x- x_b)^2 + (y- y_b)^2 = d_{bi}{}^2$$
$$(x- x_c)^2 + (y- y_c)^2 = d_{ci}{}^2$$

FIG. 2

FIG. 3

FIG. 4

FIG. 5

FIG. 6

FIG. 7

Europäisches
Patentamt

European
Patent Office

Office européen
des brevets

## EUROPEAN SEARCH REPORT

Application Number

EP 21 18 5843

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y<br>A | US 2020/390359 A1 (ALHATHAL MESHAL [US] ET AL) 17 December 2020 (2020-12-17)<br>* abstract *<br>* paragraph [0021] *<br>* paragraph [0031] *<br>* paragraph [0035] - paragraph [0039] *<br>* paragraph [0043] - paragraph [0053] *<br>* paragraph [0063] - paragraph [0079] *<br>* figures 1,2,4,6-8 *<br>----- | 14<br>15<br>1,13 | INV.<br>A61B5/00<br>A61B5/389 |
| Y<br><br><br><br><br><br><br><br><br><br><br><br><br>A | WILLEM MONSTER A ET AL: "Surface electromyogram potentials of motor units; Relationship between potential size and unit location in a large human skeletal muscle",<br>EXPERIMENTAL NEUROLOGY, ELSEVIER, AMSTERDAM, NL,<br>vol. 67, no. 2,<br>1 February 1980 (1980-02-01), pages 280-297, XP026243903,<br>ISSN: 0014-4886, DOI:<br>10.1016/0014-4886(80)90230-7<br>[retrieved on 1980-02-01]<br>* abstract *<br>* page 280 - page 291 *<br>* figures 1-5 *<br>----- | 15<br><br><br><br><br><br><br><br><br><br><br><br><br>1,7,8,<br>13,14 | |
| | -/-- | | TECHNICAL FIELDS SEARCHED (IPC)<br><br>A61B |

The present search report has been drawn up for all claims

| Place of search | Date of completion of the search | Examiner |
|---|---|---|
| The Hague | 19 November 2021 | Van der Haegen, D |

EPO FORM 1503 03.82 (P04C01)

page 1 of 3

Europäisches
Patentamt

European
Patent Office

Office européen
des brevets

**EUROPEAN SEARCH REPORT**

Application Number

EP 21 18 5843

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Gydikov A ET AL: "Studying the alpha motoneurone activity by investigating motor units of various sizes", Electromyogr Clin Neurophysiol . Apr-Jun 1972;12(2):99-117, 1 April 1972 (1972-04-01), pages 99-117, XP55863401, Retrieved from the Internet: URL:http://www.ncbi.nlm.nih.gov/pubmed/4653370 [retrieved on 2021-11-19] * abstract * * page 99 - page 110 * * figures 1-6 * | 1,7,8, 13-15 | |
| A | TADASHI MASUDA ET AL: "TOPOGRAPHICAL MAP OF INNERVATION ZONES WITHIN SINGLE MOTOR UNITS MEASURED WITH A GRID SURFACE ELECTRODE", IEEE TRANSACTIONS ON BIOMEDICAL ENGINEERING, IEEE, USA, vol. 35, no. 8, 1 August 1988 (1988-08-01) , pages 623-628, XP000005699, ISSN: 0018-9294, DOI: 10.1109/10.4595 * the whole document * | 1,7,8, 13-15 | TECHNICAL FIELDS SEARCHED (IPC) |
| A | GRIEP P A M ET AL: "Calculation and registration of the same motor unit action potential", ELECTROENCEPHALOGRAPHY AND CLINICAL NEUROPHYSIOLOGY, ELSEVIER, NL, vol. 53, no. 4, 1 April 1982 (1982-04-01), pages 388-404, XP024103178, ISSN: 0013-4694, DOI: 10.1016/0013-4694(82)90004-9 [retrieved on 1982-04-01] * the whole document * | 1,7,8, 13-15 | |

-/--

The present search report has been drawn up for all claims

| Place of search | Date of completion of the search | Examiner |
|---|---|---|
| The Hague | 19 November 2021 | Van der Haegen, D |

Europäisches
Patentamt

European
Patent Office

Office européen
des brevets

# EUROPEAN SEARCH REPORT

Application Number

EP 21 18 5843

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2014/066803 A1 (CHOI CHARLES TAK MING [TW]) 6 March 2014 (2014-03-06)<br>* abstract *<br>* paragraph [0033] - paragraph [0066] *<br>* figures 1-3,5, 7A-D, 9-10B *<br>----- | 1,8, 13-15 | |
| A | US 2018/092559 A1 (WYBO CHRISTOPHER [US]) 5 April 2018 (2018-04-05)<br>* abstract *<br>* paragraph [0003] - paragraph [0006] *<br>* paragraph [0032] - paragraph [0062] *<br>* figures 1,2,6,7,9A-10B *<br>----- | 1,8, 13-15 | |

TECHNICAL FIELDS
SEARCHED     (IPC)

The present search report has been drawn up for all claims

| Place of search | Date of completion of the search | Examiner |
|---|---|---|
| The Hague | 19 November 2021 | Van der Haegen, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 18 5843

This annex lists the patent family members relating to the patent documents cited in the above-mentioned European search report.
The members are as contained in the European Patent Office EDP file on
The European Patent Office is in no way liable for these particulars which are merely given for the purpose of information.

19-11-2021

| Patent document cited in search report | | Publication date | Patent family member(s) | | Publication date |
|---|---|---|---|---|---|
| US 2020390359 | A1 | 17-12-2020 | NONE | | |
| US 2014066803 | A1 | 06-03-2014 | CN | 103654762 A | 26-03-2014 |
| | | | TW | 201408258 A | 01-03-2014 |
| | | | US | 2014066803 A1 | 06-03-2014 |
| US 2018092559 | A1 | 05-04-2018 | US | 2018092559 A1 | 05-04-2018 |
| | | | US | 2019269342 A1 | 05-09-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **J. WEISS ; L. D. WEISS ; J. K. SILVER ; D. J. DOWLING.** *Easy EMG: a guide to performing nerve conduction studies and electromyography,* 2016

- **C. J. DE LUCA ; A. ADAM ; R. WOTIZ ; L. D. GILMORE ; S. H. NAWAB.** Decomposition of Surface EMG Signals. *Journal of Neurophysiology,* September 2006, vol. 96 (3), 1646-1657